# EUROPEAN PATENT SPECIFICATION

(11) **EP 4 356 819 B1**
(45) Date of publication and mention of the grant of the patent: **03.12.2025**
(21) Application number: 22202749.2
(22) Date of filing: 20.10.2022
(51) Int. Cl.: A61B 5/00, A61B 5/0536, A61B 5/20

(54) **DEVICE FOR DETERMINING BLADDER FILLING LEVEL OF THE URINARY BLADDER OF A USER IN A NON-INVASIVE MANNER AND METHOD THEREFOR**
VORRICHTUNG ZUR NICHTINVASIVEN BESTIMMUNG DES BLASENFÜLLSTANDS DER HARNBLASE EINES BENUTZERS UND VERFAHREN DAFÜR
DISPOSITIF DE DÉTERMINATION NON INVASIVE DU NIVEAU DE REMPLISSAGE DE VESSIE DE L'UTILISATEUR PAR LA VESSIE ET PROCÉDÉ ASSOCIÉ

(43) Date of publication of application: 24.04.2024
(73) Proprietor: inContAlert GmbH, 90619 Trautskirchen (DE)
(72) Inventor: Fechner, Pascal, 95352 Marktleugast (DE); Lockl, Jannik, 90619 Trautskirchen (DE); Ruhland, Nicolas, 92655 Grafenwöhr (DE); Zürl, Tristan, 95497 Brandholz (DE)
(74) Representative: Hoffmann Eitle

(56) References cited:
- WO-A1-2022/182794
- DE-A1- 10 315 863
- DE-A1- 102011 108 252
- DE-A1- 102019 207 154
- US-A1- 2006 276 712
- US-A1- 2018 263 546
- DUNNE EOGHAN ET AL: "Supervised Learning Classifiers for Electrical Impedance-based Bladder State Detection", SCIENTIFIC REPORTS, vol. 8, no. 1, 29 March 2018 (2018-03-29), XP093030798, Retrieved from the Internet <URL:https://www.nature.com/articles/s41598-018-23786-5> DOI: 10.1038/s41598-018-23786-5
- FECHNER PASCAL ET AL: "Near-Infrared Spectroscopy for Bladder Monitoring: A Machine Learning Approach", ACM TRANSACTIONS ON MANAGEMENT INFORMATION SYSTEMS, 22 September 2022 (2022-09-22), XP093030828, Retrieved from the Internet <URL:https://web.archive.org/web/20221203062200if_/https://dl.acm.org/doi/pdf/10.1145/3563779> [retrieved on 20230310]

## Description

### Technical field

The present disclosure relates to a non-invasive device for determining a filling level, specifically for determining the filling level of the urinary bladder of a user, and to a computer-implemented method for producing a trained model capable of determining the filling level of the urinary bladder of a user.

### Technical background

A non-invasive measuring/determining device for determining a bladder filling level or a fluid retention (wherein the device is capable of sending a signal to indicate when a predefined/critical level is reached and/or when a predefined timespan is remaining until the predefined level is reached) can, e.g., help urinary incontinence sufferers to figure out a filling level of the urinary bladder. All forms of urinary incontinence have in common that, once the disorder reaches a certain level of severity, they can force the affected person to use absorbent aids (e.g., diapers, pads) or drainage aids (e.g., catheters), thus considerably limiting the affected person's quality of life.

Certain individuals cannot independently protect themselves from bladder overflow due to a lack of knowledge about overfilling, such as persons with a neurogenic bladder (e.g., para- or tetraplegics, multiple sclerosis, Parkinson's disease, stroke, spina bifida patients, or patients with a neobladder). However, in addition to incontinence symptoms (e.g., dribbling urine), an overfilling of the bladder or a vesicoureteral reflux can also occur, increasing a risk of unphysiological reflux of urine into the renal pelvis, which is detrimental to health.

In this regard, near-infrared spectroscopy (NIRS) can be a non-invasive technique used to collect data that allows to estimate the filling level of the bladder by using the absorption, reflection and scattering properties of organic tissue. In NIRS, an emitted radiation of light from an infrared radiation source is irradiated into the tissue of interest, and the intensity of the outgoing radiation is measured at another location. The ratio of the signal amplitudes provides information about the extinction or attenuation and scattering of the light beam. Different tissues have different extinction properties (or extinction coefficients), making it possible to provide information about the concentration of different tissue substances or substance groups in the illuminated area of the body.

The water concentration in human tissue can provide information about a variety of processes in the human body, such as muscle parameters in athletes or information about the brain. Here, Molavi et al. (DOI: 10.1109/TBCAS.2013.2272013) describe a wireless and portable device for non-invasive optical observation of bladder filling status using NIRS. A device using NIRS for measuring fluid retention in the urinary bladder of a user is known from DE 10 2019 207 154 A1.

Also, electrical impedance spectroscopy (EIS) can be another non-invasive sensing technique for monitoring the condition of the human bladder. Liao & Jaw (DOI:10.1111/j.1447-0756.2010.01487.x) propose an approach for monitoring of the bladder fullness with bioimpedance measurements and Rodas et al. (DOI: 10.1007/978-981-10-9038-7_172) perform an estimation of emptying the urinary bladder of paraplegics and elderly people based on bioimpedance and hypogastric region temperature.

Also, electrical impedance tomography (EIT) can be another non-invasive imaging technique for monitoring the condition of a human bladder. One such method is proposed by Khambampati et al. (DOI: 10.1109/TENCON.2018.8650499), wherein the forward problem of EIT is solved using a boundary element method while the inverse problem of estimating the size and shape of bladder is done with the Levenberg-Marquardt method. DE 103 15 863 A1 relates to a belt with electrodes for performing EIT. Also, Noyori et al. (DOI: 10.1109/IEEECONF49454.2021.9382658) propose an approach for performing urine volume estimation by EIT with fewer electrodes. DE 10 2011 108 252 A1 relates to an EIT method using four or more electrodes.

From WO 2022/182 794 A1 a device using bio-impedance spectroscopy to determine one or both of bladder volume and total body water is known. US 2018/0 263 546 A1 to a method, system and a device for non-invasive detection of urine flow from the bladder into the kidney(s). US 2006/0 276 712 A1 relates to a method for monitoring bladder function in an animal having a bladder provided with a light shield apparatus and filter apparatus for near infrared spectroscopy (NIRS) bladder monitoring. Also, by Dunne Eoghan et al. (DOI: 10.1038/s41598-018-23786-5) a method for supervised learning classifiers for electrical impedance-based bladder state detection is known.

### Summary of the Invention

### Technical problems

Each of NIRS, EIS and EIT as discussed above provide advantages and drawbacks associated with each of the underlying technologies. E.g., differences in skin coloring (e.g., due to pigmentation, blood vessels, sex, or genetics) and body anatomy (e.g., fat, bone and muscle) make it challenging to acquire accurate estimations of the filling level of the urinary bladder. Also, if not positioned correctly on the body of the user, the device may not provide readings pertaining to the urinary bladder, but other organs located in its vicinity. Also, changes in size of other organs located in the vicinity of the urinary bladder and movement or changes in orientation of the human body may cause a shift or a deformation of the urinary bladder, which would further decrease the estimation accuracy.

Therefore, it is an object of the present disclosure to address drawbacks of the known techniques and to provide a device and method to accurately and reliably determine the filling level of the urinary bladder of a user in a non-invasive manner.

### Technical Solution

The subject-matter of the independent claims solves these technical problems. The dependent claims describe further preferred embodiments, and the subsequent description explains preferred modes for carrying out the present invention.

Specifically, a non-invasive device for determining a filling level of the urinary bladder of a user according to claim 1 (hereon also referred to as "determining device"), a computer-implemented method for producing a trained model capable of determining a filling level of the urinary bladder of a user according to claim 9 and a system for determining a filling level of the urinary bladder of a user according to claim 13 (hereon also referred to as "determining system") solve these technical problems.

Herein, a combination of the measuring techniques of NIRS, EIS and/or EIT is implemented into a single device that uses the techniques in a complementing manner. Specifically, NIRS, EIS and EIT are selectively combined to assure that the measurements provided by each of the techniques have been obtained when the corresponding technique operates in its best measuring environment. Thereby, the accuracy of the determining can be improved despite some of the measuring techniques not operating in their optimal measuring/working range.

Further, through the interaction of the individual measurement techniques and the parameters thus determined, it becomes possible to indicate to the user if the measuring device is not correctly positioned and to give feedback to the user on how to reposition the device to achieve a better measurement quality and thereby a better determining of the filling level of the urinary bladder.

Further, a disturbance variable detection can be used to detect that the measurement by one of the measurement techniques has changed in accuracy (e.g., due to an external influence or noise). Based thereon, the measurements from different measurement techniques can be (selectively or preferentially) used in a manner to improve the determining accuracy as a whole (i.e., with respect to the determining device and not with respect to each of the measurement techniques).

Further, by allowing EIS and EIT to share the same hardware, a compact size of a more accurate determining device is obtained. Further, by using a model trained on the basis of measurement signals obtained from the determining device, a determination mechanism is obtained that can reliably determine the urinary filling level of the urinary bladder based on one or more measurement signals. This determination is enabled despite the complexity of integrating and evaluating multiple and potentially conflicting measurement signals originating from different measurement techniques. As a preferred further embodiment, EIT and/or peripheral measurements can be used to further improve the determination.

### Brief summary of drawings

Fig. 1 is an illustration of the core components of a non-invasive determining device according to an embodiment of the present disclosure.
Fig. 2 is an illustration of a process to train a model for bladder monitoring according to an embodiment of the present disclosure.
Fig. 3 is an illustration of a front view of a non-invasive determining device according to an embodiment of the present disclosure.
Fig. 4 is an illustration of a rear view of a non-invasive determining device according to an embodiment of the present disclosure.
Fig. 5 is an exemplifying schematic of an amplifying circuit for the light measuring unit according to an embodiment of the present disclosure.
Fig. 6 is an exemplifying schematic of a driver circuit for the light source according to an embodiment of the present disclosure.
Fig. 7 is an exemplifying schematic of an analog front end for the electrodes of the voltage measuring unit according to an embodiment of the present disclosure.
Fig. 8 is an exemplifying functional diagram of an IC of the analog front end for the electrodes of the voltage measuring unit according to an embodiment of the present disclosure.
Fig. 9 is an exemplifying circuit of the light measuring unit according to an embodiment of the present disclosure.
Fig. 10 is an exemplifying illustration of a belt with electrodes to be connected to the determining device according to an embodiment of the present disclosure.
Fig. 11 is an exemplifying illustration of dimensions of the belt with electrodes to be connected to the determining device according to an embodiment of the present disclosure.

### Detailed description

The present disclosure is described in detail below with reference to the drawings.

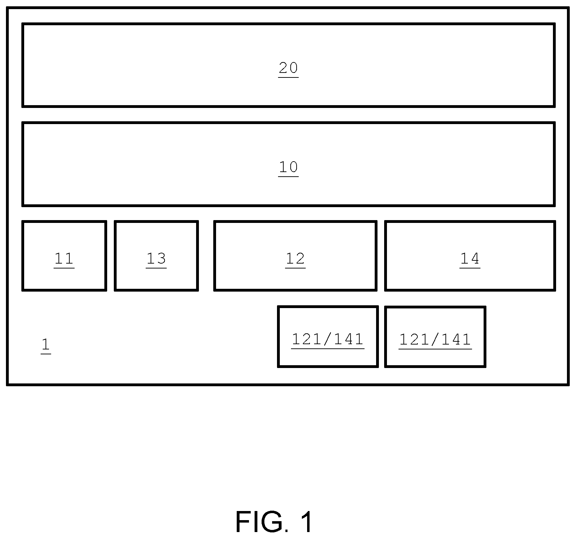

Fig. 1 illustrates a non-invasive device 1 for determining a filling level of the urinary bladder of a user (i.e., a "determining device") according to an embodiment of the present disclosure. This determining device 1 comprises a control unit 10, a light source 11, a current source 12, a light measuring unit 13, a voltage measuring unit 14 and an evaluation unit 20. Here, the determining device 1 may be adapted to rest on the body (or body surface) of a user to measure or determine the filling level of (or fluid retention in) the urinary bladder (hereon also referred to as just "filling level").

The light source 11 is adapted to irradiate light into a region of the user's body covering the pelvic region. Herein, the region of the user's body covering the pelvic region (hereon also referred to as "the region of the user's body") may be a region below the stomach of a human. The region of the user's body may be at a location coverable by a wearable item **(e.g.,** a T-shirt, underwear or a belt), allowing the determining device 1 or part/s thereof to be attached to or embedded within the wearable item.

For the generation of light that can penetrate human tissue sufficiently deep, but also react very sensitively to changes in it, in principle any radiation source or light source emitting in an optical window **(i.e.,** wavelength range at which a slight attenuation of the radiation in organic tissue occurs) can be used. However, to enable comfortable and safe use of the determining device 1 aimed at personal use by users/patients, further requirements should be met. Specific requirements may be a narrow emission spectrum with a single maximum, mechanical robustness, chemical robustness in contact with human skin or its secretions, non-hazardous in eye contact with the emitted radiation, non-hazardous in skin contact with the radiation source, high efficiency, low component costs. Here light emitting diodes (LEDs) may be used as their brightness can be accurately controlled using a variable current source **e.g.,** implemented using an operational amplifier. Further LEDs may be chosen that emit a narrow spectrum of light surrounding a single wavelength. Thereby, the light can be tuned according to the above-mentioned requirements for penetration and attenuation.

As a modification to the present embodiment, the light irradiated by the light source 11 may be near-infrared (NIR) light, preferably with a wavelength of at least 600 nm and at most 1100 nm and more preferably with a wavelength of at least 830 nm and at most 975 nm. The light source may comprise a plurality of light emitting elements (or LEDs) configured to emit NIR light of 775 nm, 855 nm, 880 nm, 940 nm, 950 nm, 970 nm, 975 nm, 1020 nm and/or 1050 nm. That is each of the plurality of light emitting elements may emit light of multiple wavelengths or different ones of the plurality of light emitting elements may emit light of different wavelengths. Both approaches may however be used to enable the light source 11 to irradiate light covering a predetermined spectrum of light.

The current source 12 is adapted to output and pass a current through the region of the user's body using at least two (or more) electrodes 121 (hereon also referred to as "current electrodes"). The current electrodes 121 may be provided as part of the determining device 1 or may be provided in a detachable manner. When the current electrodes 121 can be detached from the determining device 1, the determining device 1 may comprise terminals, sockets, connectors or the like for forming an electrical connection between the current electrodes 121 and the current source 12.

For the generation of a current that can safely pass-through human tissue either direct current (DC) or alternating current (AC) may be used. DC allows the determination of a resistive (bio)impedance. AC allows the determination of a resistive (bio)impedance and of a capacitive/inductive component of the (bio)impedance. In either case, the strength or peak strength of the current may be chosen so as to prevent damage to the human tissue (see also DIN EN 60601-1). Herein, it is preferrable to use AC since continuous DC (even at lower currents) entails the danger of causing permanent tissue damage if not adequately regulated. To prevent any DC from being outputted by the electrodes, one or more capacitors may be connected in series with each electrode.

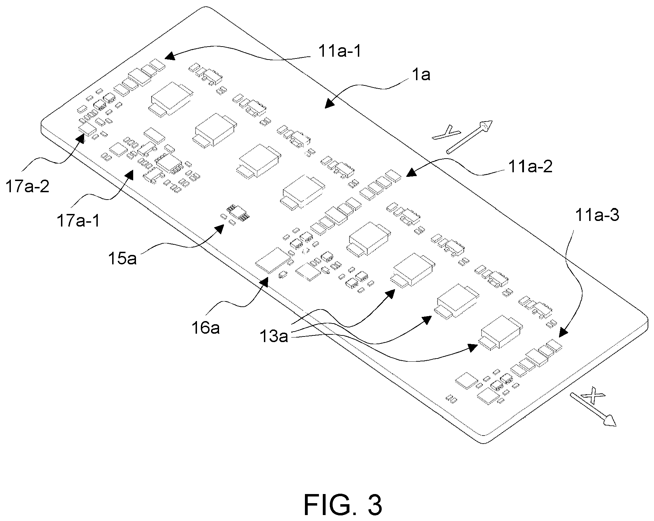

The control unit 10 is adapted to output, to the light source 11, a first control signal (or a lighting control signal) matched to the user, for controlling the light irradiated by the light source 11, and to output, to the current source 12, a second control signal (or a current control signal) matched to the user, for controlling the current output by the current source 12. A first power supply unit (e.g., unit 17a-1 in Fig. 3) may be used as a power supply for the light source 11. A second power supply unit (e.g., unit 17a-2 in Fig. 3) may be used as a power supply for the current source 12 to apply a current via the current electrodes 121. Herein, the first power supply unit and the second power supply unit may be separate units or may be combined into a single power supply unit.

Based on the power output capabilities of the one or more power supplies of the determining device 1, the two control signals may be adapted to activate the light source 11 and the current source 12 in an alternating manner (i.e., quasi-simultaneously). Alternatively, they may be activated at the same time (i.e., simultaneously). A further advantage of activating the light source 11 and the current source 12 quasi-simultaneously is that unwanted interference between signals can be avoided.

To implement the control signals matched to the user, the control unit 10 may comprise, e.g., a first register to control the amount of current flowing through the light source 11 (e.g., its LEDs) and a second register to control the amount of current outputted by the current source 12.

When the light source 11 is constituted by multiple LEDs, the control unit 10 may comprise multiple first registers, i.e., one for each LED, to indicate the amplitude of the current for each LED. Similarly, when the current source 12 provides current to multiple current electrodes 121, the control unit 10 may comprise multiple second registers, i.e., one for each current electrode 121, to indicate the amplitude of the current (and direction of the current) for each current electrode 121. A current of an amplitude value corresponding to the value stored in the register of the control unit 10 may be obtained by applying a PWM signal with adjustable frequency and duty ratio or an operational amplifier based current source circuit. By adjusting both frequency and duty ratio of the PWM signal or the amplitude and frequency of the inputted signal of the amplifier circuit the currents can be matched to the user's anatomy.

The control signals outputted by the control unit 10 may be PWM signals or sine signals applied to drive circuits of the light source 11 and of the current source 12. Alternatively, if the drive circuits are digitally controllable ICs, a communication interface (e.g., I2C, SPI, CAN, one-wire or similar protocols) may be used to communicate the PWM or waveform configurations from the control unit 10 to the driver circuit ICs.

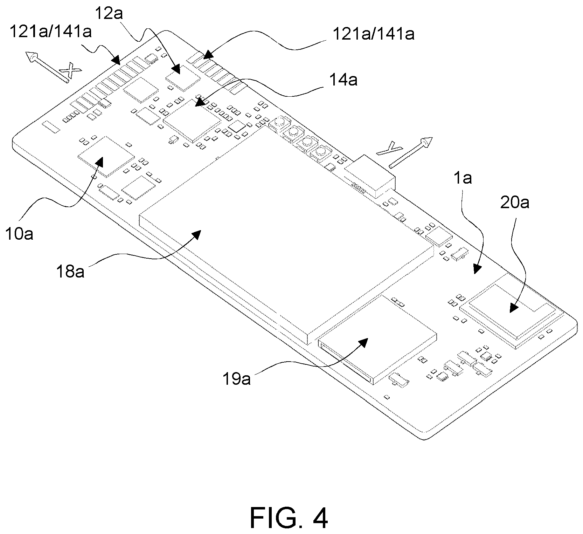

The light measuring unit 13 is adapted to measure the light emitted from the light source 11 and light reflected from organs of the user's body, and to output a first measuring signal (or lighting measurement signal) corresponding to the measured light intensity. Herein, and as further explained below in reference to Fig.3 and Fig. 4, the light measuring unit 13 may be a photo detector, preferably an IR-detector or IR-photodiode sensitive for a broad spectrum of wavelengths ranging from 600nm to 1100nm with a maximum sensitivity at 870 nm with a detection surface of 7 mm² and a detection sensitivity of 46 V/(mW/cm²).

The voltage measuring unit 14 is adapted to measure a voltage based on the current passed through the region of the user's body using at least two (or more) electrodes 141 (hereon also referred to as "voltage electrodes") resting against the user's body, and to output a second measurement signal (or voltage measurement signal) corresponding to the measured voltage.

It is worth noting that, the current electrodes 121 and the voltage electrodes 141 may be the same electrodes. That is to say, at least two electrodes are required by the determining device 1. That is, the current source 12 and the voltage measuring unit 14 may share common electrodes (or terminals, connectors, sockets or the like). Put differently, and as indicated in Fig. 1, the two electrodes 121/141 may perform both the function of current electrodes 121 and voltage electrodes 141.

To simplify the circuitry, it is preferable that more than two electrodes 121/141 are provided allowing the current output and voltage measurement to be performed using different pairs of electrodes, respectively, or sharing only one common electrode. **E.g.,** this common electrode may be a common ground shared by the current source 12 and the voltage measuring unit 14. Thereby, the current source 12 may output and pass the current through the region of the user's body, and the voltage measuring unit 14 may measure the voltage based on the current passed through the region of the user's body, but the number of components is reduced.

Herein, the light measuring unit 13 and the voltage measuring unit 14 may be adapted to perform measuring repetitively at a predetermined (excitation) frequency. This frequency may be at least 10 kHz and at most 200 kHz and is preferably 100 kHz. The sampling frequency is at least by a multiple of two greater than the corresponding excitation frequency, **e.g.,** 800 kSPS for the voltage measuring unit and 500 kSPS per detector. Herein, the light source 11 and light measurement unit 13 may enable NIRS and the current source 12 and the voltage measurement unit 14 may enable EIS.

The evaluation unit 20 is adapted to determine the filling level of the urinary bladder based on the first measurement signal and/or the second measurement signal (i.e., the lighting measurement signal and/or the voltage measurement signal).

As a modification to the present embodiment, the determining device 1 may further comprise a peripheric measuring unit adapted to measure peripheric properties including acceleration forces, rotational movements, temperature and/or humidity, and to output a third measurement signal (or peripheric measurement signal) corresponding to the peripheric properties. In this case, the evaluation unit may be further adapted to determine the filling level of the urinary bladder based on the first measurement signal, the second measurement signal and/or the third measurement signal (i.e., the lighting measurement signal, the voltage measurement signal and/or the peripheric measurement signal). That is to say, the determination may be based on at least one of the first measurement signal, the second measurement signal and the third measurement signal.

As a modification to the present embodiment, the determining device 1 may comprise at least eight current electrodes 121 that are distributed over the region of the user's body (possibly including the previously mentioned two current electrodes 121). Herein, the second control signal may control the current source 12 to pass the current through the region of the user's body using a varying selection of two or more current electrodes 121 of the at least eight current electrodes 121.

It is preferrable that the plurality of current electrodes 121 are distributed in a circumferential manner to cover close to 360 degrees of the user's body. This circumferential distribution may be achieved by providing the electrodes as part of a wearable or portable (e.g., a belt, bandage, T-shirt, underwear or the like worn by the user, or an adhering means such as a plaster, suction cup or the like) that is worn by or applied to the user. By use of such a wearable or portable the current electrodes 121 may be distributed over the user's body. By providing the current electrodes 121 as part of a wearable, proper distribution may be assured since the wearable is typically designed to be worn in a specific way. As a result, even when the determining device 1 is not properly positioned on the user's body, since at least some of the current electrodes 121 will be positioned over the region of the user's body covering the urinary bladder EIS may still be performed for determining the filling level of the urinary bladder of the user.

As a modification to the present embodiment, the voltage measuring unit 14 may be adapted to measure voltages based on different selections of two or more voltage electrodes 141 (that may be the eight current electrodes 121), and to output second measurement signals corresponding to the measured voltages. Herein the evaluation unit 20 may be adapted to compute an impedance distribution of a cross section of the user's body (or an EIT measurement) based on the second measurement signals, and to determine the filling level of the urinary bladder based on the first measurement signal, the second measurement signal, the third measurement signal, and/or the impedance distribution (i.e., the lighting measurement signal, the voltage measurement signal, the peripheric measurement signal and/or the EIT measurement). That is to say, the determination may be based on at least one of the first measurement signal, the second measurement signal, the third measurement signal and the impedance distribution.

Thereby, the same electrodes 121/141 that are used for EIS may also be used for EIT. To improve the resolution of EIT, the number of electrodes may be larger than eight, e.g., 16, 32, or more. Additionally, multiple layers of electrodes 121/141 may be provided to not limit the EIT to a single cross section of the user's body. Using electrodes from different layers may also allow EIT measurements over inclined cross sections of the user's body. Therefore, even if the determining device 1 is positioned too high or too low on the user's body or at an angle, EIS and EIT can still be performed and the filling level of the urinary bladder of the user can still be determined based thereon.

As a modification to the present embodiment, the evaluation unit 20 may be configured to select the first measuring signal and/or the second measuring signal (i.e., the lighting measurement signal and/or the voltage measurement signal) based on a (selection) parameter, wherein the parameter indicates the reliability or usability of the light measuring unit 13 and/or the voltage measuring unit 14. Further, when the peripheric measuring unit 15 is provided, the evaluation unit 20 may be configured to select the first measuring signal, the second measuring signal and/or the third measuring signal (i.e., the lighting measurement signal, the voltage measurement signal and/or the peripheric measurement signal) based on the parameter, wherein the parameter indicates the reliability or usability of the light measuring unit 13, the voltage measuring unit 14 and/or the peripheric measuring unit 15. Further, when the impedance distribution is computed, the evaluation unit 20 may be configured to select the first measuring signal, the second measuring signal, the third measuring signal and/or the impedance distribution (i.e., the lighting measurement signal, the voltage measurement signal, the peripheric measurement signal and/or the EIT measurement) based on the parameter.

That is to say, the evaluation unit 20 may select NIRS, EIS, EIT and/or periphery measurements based on a parameter, representing the reliability or usability of the underlying measuring technique. Herein, the parameter may be a single value (e.g., a statistical value such as a weighted average), a plurality of values (e.g., a set of thresholds or of flags for determining whether any one of the units is sufficiently reliable or usable for the determining) or a matrix (e.g., a state matrix) representing the reliability or usability of the individual measuring techniques.

Reliability and usability are terms summarizing whether the corresponding unit operates close to or in its optimal measuring/operating range. Therefore, the reliability or usability of the individual measuring techniques may be determined based on the first measuring signal, the second measuring signal, the third measuring signal and/or the impedance distribution. Thereby, the parameter can indicate the present situation or state of the user and/or of the determining device 1. These situation or state of the user and/or of the determining device 1 are the predominant influence on the reliability or usability of the light measuring unit 13, the voltage measuring unit 14 and the peripheric measuring unit 15.

The parameter may be impacted by the posture of the user, such that, if the third measuring signal (i.e., the peripheric measurement signal) indicates that the user is seated, the parameter may indicate that NIRS is less reliable or useful for determining the filling level of the urinary bladder compared to EIS and/or EIT. Therefore, the evaluation unit 20 may select only the voltage measuring unit 14 and/or the peripheric measuring unit 15 for the determining. Alternatively, for example, if the third measuring signal indicates that the user is standing or walking, the parameter may indicate that NIRS is more reliable or useful for determining the filling level of the urinary bladder compared to EIS and/or EIT. Therefore, the evaluation unit 20 may select only the light measuring unit 13 and/or the peripheric measuring unit 15 for the determining.

The parameter may be impacted by biological aspects of the user **(e.g.,** skin color, skin pigmentation, skin resistance/conductivity, sex, age, weight, height, body temperature etc.) since they may also have an impact on the reliability or usability of the individual measuring techniques. For example, a high degree of adiposity may hinder the penetration of light into the user's body. Therefore, the parameter may indicate that (for the specific user) EIS and EIT are preferrable for determining the filling level of the urinary bladder compared to NIRS. Therefore, the evaluation unit 20 may select only the voltage measuring unit 14 and/or the peripheric measuring unit 15 for the
determining.

That is to say, the parameter may be computed based on the outputs or measurements from the light measuring unit 13, the voltage measuring unit 14 and/or the peripheric measuring unit 15 to indicate the reliability or usability of the individual units, allowing the evaluation unit 20 to selectively choose the unit or combination of units for the determining, that yields the most reliable and accurate determining result. Furthermore, the evaluation unit 20 may deliberately weight the impact of each unit and/or conceivable combination on the result of the determining of the filling level, according to the reliability and accuracy of the units.

By the above-described embodiment or any of its modification, the determining device 1 combines measuring techniques of NIRS, EIS and/or EIT into a single device that uses the techniques in a complementing manner.

Further, the parameter can be used to detect a disturbance variable for the interpretation of the measurement results that can be considered in this context. For example, when the parameter falls within a predetermined region, this may indicate the disturbance variable (e.g., like swelling, discoloration, sweat, or perspiration) as noise, causing the measurements to change in accuracy. Information about the disturbance variable (specifically when classifying its cause or origin) can then be used by the unit performing the measuring to minimize the impact of the disturbance variable.

Hence, as a modification to the present embodiment, the control unit 10, 10a may be configured to adjust the first control signal and/or the second control signal based on the parameter. This means, the control unit 10, 10a may adjust the outputs of the current source 12, 12a and/or light measuring unit 13, 13a to minimize the impact of the disturbance variable. The information about the disturbance variable may be provided by the user (e.g., through a computer or smartphone communicating with the determining device 1), or may be inferred from the parameter (i.e., the reliability or usability of the individual units) itself. For example, sweat or perspiration has a larger impact on EIS and EIT compared to NIRS, whilst swelling or discoloration has a larger impact on NIRS compared to EIS and EIT (e.g., sweat or perspiration reduces the skin's resistance improving the sensitivity and reliability of EIS and EIT).

Further, the parameter may be used to assess whether the positioning of any one of the sensing units of the determining device 1 (i.e., light source 11, the electrodes 121 of the current source 12, the light measuring unit 13 and the electrodes 141 of the voltage measuring unit 14) on the user's body needs to be corrected. For example, if the parameter indicates that too few or none of the sensing units is reliable or usable enough to perform the determining, feedback can be given to the user to help replace one or more sensing units on the body in such a way that optimal measurement conditions are created.

Hereon, reference is made to a "position of a sensing unit" for conciseness and brevity, to abbreviate writing the "position of the light source 11, the electrodes 121, 141, and/or the light measuring unit 13".

Also, as a modification to the present embodiment, the evaluation unit 20, 20a may be configured to determine an accuracy of a positioning (or placement) of a sensing unit on the body of the user by using the first measuring signal, the second measuring signal, the third measuring signal and/or the impedance distribution (i.e., the lighting measurement signal, the voltage measurement signal, the peripheric measurement signal and/or the EIT measurement), wherein the sensing unit/s is/are the light source 11, the electrodes 121, 141, and/or the light measuring unit 13. This accuracy determination may be performed in addition to or instead of the position assessment based on the (selection) parameter.

Herein, to determine the accuracy of the positioning of the sensing unit/s, the evaluation unit 20, 20a may be configured to compare a (predetermined, prerecorded or initial) reference measurement obtained from the light measuring unit 13, 13a, the voltage measuring unit 14, 14a and/or the peripheric measuring unit 15a, 16a with the first measuring signal, the second measuring signal, the third measuring signal and/or the impedance distribution.

That is to say the reference measurement may be obtained by taking one (or more) measurements after (i.e., preferably immediately after) an initial application of the determining device 1 and its sensing unit/s to the body of the user. Because the initial application by the user is usually the most careful application according to application instructions or user manual, a proper positioning of the sensing unit/s is most likely. Alternatively, the initial application may be performed by a medical professional to guarantee a correct positioning of the sensing unit/s. Therefore, the reference measurement obtained by the light measuring unit 13, 13a and/or the voltage measuring unit 14, 14a after this initial application may be used for the comparison. By comparing subsequent measurements to this initial measurement, a change in positioning can be detected and fed back or notified to the user. Herein, the change in positioning relative to an optimal positioning may be due to movement or may be due to a mispositioning of the sensing unit/s (or the determining device 1) by the user.

It is worth noting, that the reference measurement may be obtained during an application subsequent to the initial application. For example, if the initial application was performed by the user and one of the subsequent applications is performed by a medical professional, the reference measurement may be obtained after **(i.e.,** preferably immediately after) the subsequent application. Prior to this subsequent application, a change in positioning may be fed back or notified to the user based on a less optimal reference measurement only, or feedback or notifications may only be enabled once a medical professional has performed the application at least once.

Also, to determine the accuracy of the positioning of the sensing unit/s, the evaluation unit 20, 20a may be configured to:
(1) detect an anatomic marker based the first measuring signal, the second measuring signal, the third measuring signal and/or the impedance distribution;
(2) determine a position of the sensing unit/s on the body of the user from the location to the anatomic marker; and
(3) compare the determined position with a predetermined ideal position.

Herein, the atomic marker may be an organ, bone, tissue composition, blood vessel etc. that is typically found in the same location within the pelvic region of a human. When analyzing the measurements, the anatomic marker can be detected in the measurements, and from this detection, the location of the sensing unit/s on the body of the user can be deduced. For example, the shape of bladder, intestine, spine, pelvis etc. can be detected in the impedance distribution obtained from EIT. Because the location of such anatomic markers are known and similar amongst different people, and because the position of the sensing unit/s relative to its/their measurements is known, it can be deduced where on the body the sensing unit/s is/are located.

The evaluation unit 20, 20a may use algorithms therefore that are also used for "photogrammetry" to determine the location of a camera with respect to a scene based on one or more images. However, instead of using images, the evaluation unit 20, 20a may use the first measuring signal, the second measuring signal, the third measuring signal and/or the impedance distribution (i.e., the lighting measurement signal, the voltage measurement signal, the peripheric measurement signal and/or the EIT measurement).

Also, the evaluation unit 20, 20a may be configured to (i.e., additionally or alternatively) determine an accuracy of a positioning (or placement) of the determining device 1 on the body of the user, similar to the determining of the accuracy of the positioning of the sensing unit/s as described above. For example, if the sensing unit/s is/are part of the determining unit 1 or is/are located in close proximity to the determining device 1, the above mutually applies to the determining of the accuracy of the position of the determining device 1. This means, in the above, the phrase "position of the sensing unit/s" may be replaced with "position of the determining device 1".

A computer-implemented method for producing a trained model capable of determining a filling level of the urinary bladder of a user is another embodiment of the present disclosure.

The method comprises applying a determining device 1 according to an embodiment of the present disclosure to the region of the user's body. Herein, the determining device 1 used for the training of the model may be the same determining device 1 that is deployed **(i.e.,** that is used to determine the filling level of the urinary bladder of the user). It is, however, also possible to use a different determining device 1 exclusively for the training of the model and to extract the trained model from this determining device 1 to provide this stored mode to a different determining device 1 that is deployed. Herein, the trained model may be provided by use of a computer or smartphone communicating with the determining device 1 that is deployed.

The method further comprises obtaining time series data based on the first measurement signal, the second measurement signal, the third measurement signal and/or the impedance distribution **(i.e.,** the lighting measurement signal, the voltage measurement signal, the peripheric measurement signal and/or the EIT measurement). Herein, which of the measurement signals and impedance distribution may be obtained as the time series data, depends on the capabilities of the determining device 1 used for training. That is, if the determining device 1 is only capable of NIRS and EIS, only the first measurement signal and/or the second measurement signal **(i.e.,** the lighting measurement signal and/or the voltage measurement signal) may be used.

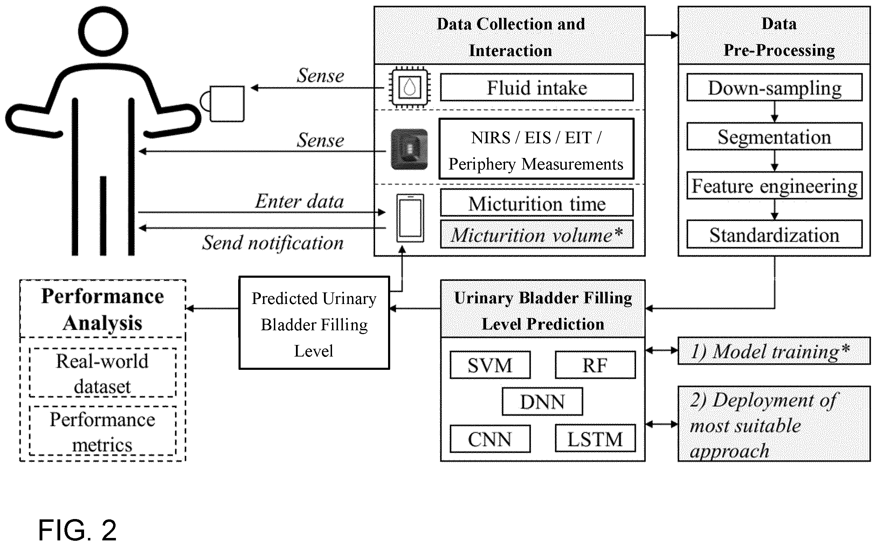

The method further comprises obtaining a notification of a predetermined event. Herein, the notification may be provided by the user **(e.g.,** by pushing a button of the determining device 1) to indicate the predetermined event. If the predetermined event is a micturition, then the notification is of a moment of time of the micturition. The predetermined event may also be the occurrence of an urgency to urinate, prior to the micturition. The notification may also be provided by use of a computer or smartphone (e.g., as illustrated in Fig. 2). Thereby, the latest datapoints of the obtained time series data may be used as a representation of a filling level of the urinary bladder. This filling level may be close to the maximum level but is at least indicative of a level at which a micturition has taken place.

It may be preferrable to obtain the filling level as a volume (e.g., by estimation, by monitoring a series of drinking events or by measuring the micturated fluid), to improve the prediction described later. This is, however, not necessary (see asterisk in Fig. 2 next to micturition volume). Only the notification of the predetermined event providing (e.g., a time micturition time in Fig. 2) is needed.

The notification may also be provided by the determining device 1 itself. E.g., when a predetermined or characteristic change of the obtained time series (e.g., indicative of a micturition) is detected by the determination device 1, the determination device 1 may trigger the notification of the predetermined event. That is to say, the user needs not provide the notification, but it may be provided automatically.

The method further comprises based on the time series data and the notification of the predetermined event. As a modification to the present embodiment the method may further comprise labeling a moment of time corresponding to a micturition as the notification of the predetermined event. The amount of urine released during the micturition may be referred to as the micturition volume or amount. This micturition volume is indicative of the latest filling level of the urinary bladder just before micturition. Therefore, the micturition amount may be used to correct the estimated filling level. Alternatively, or in addition thereto, clinical measurements may be performed to estimate the filling level **(e.g.,** an X-ray, ultrasonic or bladder pressure analysis performed by a health care professional).

As a modification to the present embodiment, the filling level may be indicative of the filling level of the urinary bladder **(i.e.,** the terms may be used interchangeably in this description) by use of a classification scheme. Herein, the classification scheme may be a binary classification, a multi-level classification, a percentage classification and/or a volumetric classification of the filling level of the urinary bladder. That is to say, that the filling level may be an indication of the actual filling level of the urinary bladder of the user, wherein such an indication may be: a binary classification of the filling level (e.g., full or empty); a multi-level classification of the filling level (e.g., a scale from 0 representing empty to 10 representing full); a percentage value (e.g., 0 % representing empty to 100 % representing full); a time interval estimate or time estimate until the next expected micturition (e.g., in minutes, hours, or intervals of 10, 15 or 30 minutes, or a specific time of the day); a volume of urine **(e.g.,** in 1 or ml); or similar representations/categorizations of the filling level.

Irrespective of the labeling, the method further comprises training the model to associate the time series data with the filling level, and to predict the filling level and/or a progress of the filling level based on the first measurement signal, the second measurement signal, the third measurement signal and/or the impedance distribution. By associating the time series data with the (estimated) filling level, a control system may be obtained capable of selecting adequate measurements for accurately determining the filling level. E.g., whilst a nearly empty urinary bladder may be better monitored using EIS, as the bladder fills with urine, NIRS may become more reliable. Because the reliability of the measurements is dependent on the quality of components of the determining device 1 as well as the user's anatomy, this dependence is taken into account by the combination. As a result, the training can be targeted on predicting the filling level as such, or on how the filling level progresses. This targeting may be based on the user's requirements. E.g., if a more accurate filling state is required, the training may be focused (e.g., by enforcement learning) on determining the filling level. If, however, the speed of urine accumulation is of importance, the training may be focused on determining the progress of filling level.

To provide further training data, it is preferrable to repeat this process for multiple iterations to obtain a larger dataset for training. It is also possible to continue training the model as it predicts the filling level. That is to say, after an initial training period where no prediction is performed, the trained model may be deployed (i.e., used to determine the filling level of the urinary bladder) and fine-tuned or better matched to the user, by applying continuous validation of the prediction (i.e., to provide feedback on the performance of the trained model).

If the user's urinary bladder size can be estimated, a percentage instead of a volume-based filling level may be estimated (e.g., 80 % instead of 400 ml for a 500 ml sized urinary bladder). The user's urinary bladder size may be estimated by using the largest filling level estimated, by monitoring the series of drinking events and/or by measuring the latest micturition volume. Specifically, when labeling the moment of time corresponding to the micturition, the largest micturition amount may be a most convenient estimate of the user's urinary bladder size. Also, if multiple iterations of estimating the filling level are performed, a maximum or average value of the estimated filling levels just before a micturition may be used as the estimate of the user's urinary bladder size. Similarly, if the micturition amounts are measured for multiple iterations, a maximum or average value of these micturition amounts may be used as the estimate of the user's urinary bladder size.

An example process of performing the training and deployment of a model is illustrated in Fig. 2. In the following, this exemplifying process is further explained. In Fig. 2, the model training is split into three distinct stages: (1) the data collection and interaction; (2) the data pre-processing; and (3) the filling level prediction.

In stage (1), NIRS, EIS, EIT and/or periphery measurements can be acquired, and the notification is provided. Using the determining device 1 or a device accompanying the determining device 1 (e.g., a computer or smartphone with a companion app) feedback data is obtained for the training. Therefrom, the measurements indicative of the filling level can be associated with the notification of the predetermined event. This association is performed by stage (2) and (3) that are described in the following.

In stage (2) the measurements are pre-processed to become suitable for machine learning mechanisms. Such mechanisms may include a support vector machine (SVM), a dense neural network (DNN), a convolutional neural network (CNN), a random forest (RF) algorithm or a long short-term memory (LSTM) neural network. Specifically, and after an optional (hardware based) filtering of the measurements, the pre-processing may constitute down-sampling, segmentation, feature extraction and standardization. By down-sampling, different sample rates of measuring units 13/13a are taken into account. Further a moving average (weighted or unweighted) may be used to filter extreme values and excursions in the measurements. The segmentation divides the measurement data into constant sample windows (e.g., of five minutes) to assure a fixed amount of data for the model training. Since additional contextual information can improve the robustness of the model training, the time elapsed (e.g., in seconds) since the last micturition and/or the estimated biological processing of fluid by the user may be modelled using Gaussian curves to account for the temporal component in fluid processing (e.g., the relationship between fluid intake and urine production). Then the features can be standardized, and they can be provided to the model training in stage (3).

In stage (3) known (supervised) model training is performed to arrive at a model (based on e.g., SVM, DNN, CNN, RF, LSTM, etc. and any combination thereof) trained to associate the measurement values with a filling level (e.g., in ml, as a percentage, or as a category of filling level). It is also conceivable to train multiple models based on different machine learning technologies. The filling levels predicted by each of the multiple models, an average thereof or a single filling level may be notified to the user. Initially, the user or the determining device 1 may continue providing training data regarding micturitions (e.g., the micturition time and/or the micturition volume) to continue the model training. After a predetermined training period lasting one or more micturitions (e.g., a period of up to and including five micturitions) the model may be deployed, meaning it may no longer require training data. It is however preferrable to continue providing training data to continue validating and optimizing the trained model. Thereby, changes in physiology (e.g., weight loss or changes in tissue composition) can be taken into account for post-calibration of the trained model. Also, when training multiple models as mentioned above, the model providing the best prediction may be deployed and the remaining models may be discarded. Thereby, the model training is customized to the user's body and daily routines.

As a modification to the present embodiment, the evaluation unit 20 may be adapted to use a model trained according to an embodiment of the present disclosure to determine the filling level of the urinary bladder. Thereby, the determining device 1 may implement a control system that is tuned to take into account the user's anatomy (e.g., estimated size of the urinary bladder and impact of the user's body on the measurements) and variations in manufacturing of the determining device 1 when determining the filling level of the urinary bladder of the user.

As an example, Fig. 3 and Fig. 4 illustrate the rough outlines of a circuit board that may be provided as the non-invasive determining device 1. This example device is referred to by reference number 1a.

Herein, the non-invasive determining device 1a comprises a light source 11a, a current source 12a, a control unit 10a for controlling the light source 11a and the current source 12a, a light measuring unit 13a, a voltage measuring unit 14a and an evaluation unit 20a. These units 10a, 11a, 12a, 13a, 14a, and 20a respectively correspond to units 10, 11, 12, 13, 14, and 20 of the non-invasive determining device 1, which has been explained above. Therefore, a repeated explanation of units 10a, 11a, 12a, 13a, 14a, and 20a is omitted and only further modifications to the present embodiment are explained in the following in reference to Fig. 3 and Fig. 4.

Specifically, in Fig. 3 and Fig. 4, the determining device 1a is exemplified as a printed circuit board (PCB) with components attached thereto. However, the PCB may be a rigid PCB made out of a composite material. Alternatively, the PCB may be flexible by providing hinging mechanisms between juxtaposed boards made out of the composite material or by forming the entire PCB out of a flexible film carrying the circuit's traces or tracks. Such a flexible PCB can be positioned better on the surface of the user's body.

Further, as shown in Fig. 3, the light source 11a may be constituted by three columns of LEDs 11a-1 to 11a-3. Herein, the columns of LEDs 11a-1 to 11a-3 may be distributed over a direction extending along an X-axis of the determining device 1a and may comprise at least five LEDs arranged in a direction extending along a Y-axis of the determining device 1a. Herein, the LEDs of each column of LEDs may be the same type of LED or may be different types of LED.

In the former case, when providing a plurality of LEDs, a component redundancy and a tolerance to rotation of the determining device 1a can be achieved, making the measuring and determining performed by the determining device 1a more reliable.

In the latter case (which is exemplified in Fig. 3), LEDs of different sizes may be provided. Specifically, and as exemplified in Fig. 3, for each column of LEDs, the circuit board may comprise four smaller pads (e.g., corresponding to a surface mount component size of 2.0 mm x 1.2 mm) and one larger pad (e.g., corresponding to a surface mount component size of 3.2 mm x 1.6 mm). Thereby, the middle LED of the five LEDs of each column of LEDs may be different (e.g., in wavelength, irradiation angles, power consumption and/or irradiation strength) compared to the other four LEDs of each column of LEDs. By allowing the possibility of providing a plurality of LEDs of the same type, the same advantages as described above can be achieved. In addition thereto, by providing at least one different type of LED (preferably along the center X-axis of the determining device 1a), light of different wavelengths can be emitted, to take into account light scattering and attenuation properties of different tissues and liquids within the user's body, that are dependent on the wavelength.

It is also conceivable to populate the determining device 1a with only one type of LEDs. E.g., in Fig. 3, only four LEDs may be provided on the smaller pads, and the larger LED may be omitted or vice versa. Thereby, the availability of LEDs can be taken into account and a re-designing of the circuit board becomes no longer necessary should some type and/or size of LEDs not be available.

Also, one of the columns of LEDs (e.g., the second column of LEDs 11a-2 of Fig. 3) may comprise additional LEDs (e.g., four additional LEDs in Fig. 3) extending in a direction along the Y-axis. Thereby, a mispositioning of the device along a direction of the Y-axis can be compensated.

Further, as shown in Fig. 3, the light measuring unit 13a may be constituted by a number of (e.g., eight) photo detectors 13a distributed along the direction along the X-axis of the determining device 1a.

The light source 11a and the light determining unit 13a may be positioned on the same side of the determining device 1a. This side may also face the user's body and may be brought in contact with the skin of the user's body. Thereby most of the light emitted by the light source 11a is emitted into the user's body, making sure stray light or leakage light is avoided as this would degrade measurements.

By positioning the columns of LEDs 11a-1 to 11a-3 between some of the photo detectors 13a light emitted into and scattered and/or reflected by the user's body may take different (light) transmission paths through the user's body. Thereby a wider measuring range is obtained, and measurements can be compared and/or corrected.

Also, the LEDs and photo detectors may be arranged symmetrically with respect to the center axis of the board extending in the Y-direction. Thereby, the user needs not to worry about a correct upwards/downwards orientation of the measurement device 1a since it can be rotated by 180 degrees without significantly impacting the measurement results.

The LEDs of the light source 11a may be activated simultaneously and all photo detectors of the light measuring unit 13a may measure light simultaneously, too. Thereby, a maximum amount of light and the deepest possible light penetration can be achieved. However, to save on energy and to allow obtaining of information regarding different transmission paths, the LEDs of the light source 11a may be activated individually and/or the photo detectors of the light measuring unit 13a may be activated individually. It is preferrable to perform simultaneous measuring by use of the photo detectors 13a and to activate the LEDs of the light source 11a individually.

Specifically, when activating the LEDs in Fig. 3 (i.e., the LEDs of the columns of LEDs 11a-1 to 11a-3) individually (e.g., turning them on and off one after the other, possibly in a random order), the photo detectors 13a may each provide a first measurement signal (or lighting measurement signal). Thereby, a number of (e.g., eight) first measurement signals may be provided by the photo detectors of the light detecting unit 13a in a quasi-parallel manner. These first measurement signals provide scatter, reflection and attenuation information for different transmission paths through the user's body. By activating each LED of the light source 11a individually the activation of each individual LED can be detected by all photo detectors 13a. Herein, LEDs may be activated e.g., at 10 kHz with a current amplitude from 0 to 100 mA and a resolution of 0.2 mA. As a result, a failure of an LED can be mapped to the Individual LED, and transmission paths from each LED to all photo detectors can be measured at the same time (i.e., in a quasi-parallel manner). By obtaining scattering and attenuation information for a plurality of transmission paths quickly and repeatedly, measurements can be improved and mispositioning of the measurement device 1a can be compensated. After all, the probability that some of the transmission paths cover the urinary bladder of the user is increased by the arrangement of LEDs and photo detectors.

Additionally, some of the LEDs of the light source 11a may not be activated at all, meaning that only a subset of LEDs may be used for performing the measuring. This can be beneficial if the mispositioning of the determining device 1a is significant enough, that certain transmission paths through the user's body yield essentially no information for allowing a determination of the filling level of the urinary bladder of the user. Also or alternatively, if some of the transmission paths through the user's body are associated with providing significant information regarding the filling level of the urinary bladder of the user, activation of LEDs associated with said transmission paths may be preferred over other LEDs. Thereby, unnecessary or less beneficial activation of LEDs can be avoided.

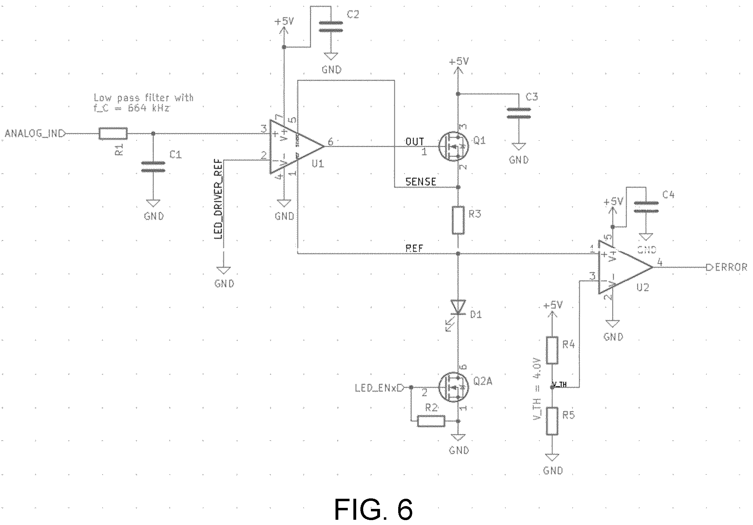

The light source (i.e., its LEDs) may be driven by a dedicated driver circuit. Fig. 6 is an illustration of an example of such a driver circuit. Herein, a transistor (e.g., using BJT or FET technology) may be used to allow driving of light sources operating at a voltage level different to that of the control unit 10. Additionally, an error signal may be obtained and as feedback for the control unit 10 to allow determining whether the activated light source (or LED) is operating correctly or whether it is defective.

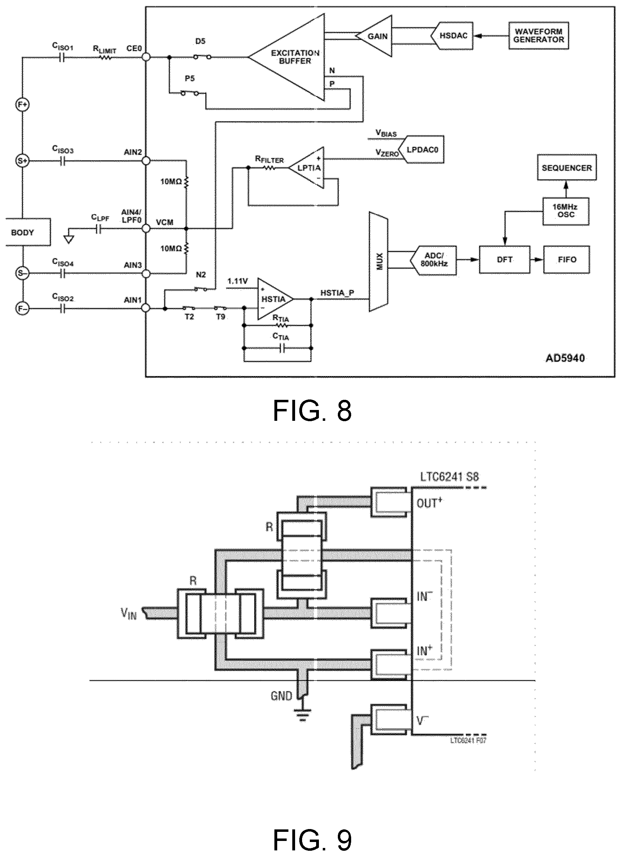

Additionally, the light source 11a and the light measuring unit 13a may be shielded from each other. This shielding may be provided by shrouds provided as part of a housing (not shown) of the determining device. Also, electromagnetic shielding may be provided, e.g., by use of ferrite cores/beads, filtering capacitors and/or guarding rings. An example of such a guarding ring is illustrated in Fig. 9. Such a guarding ring can shield leakage currents at the (inverting) terminal of an operational amplifier.

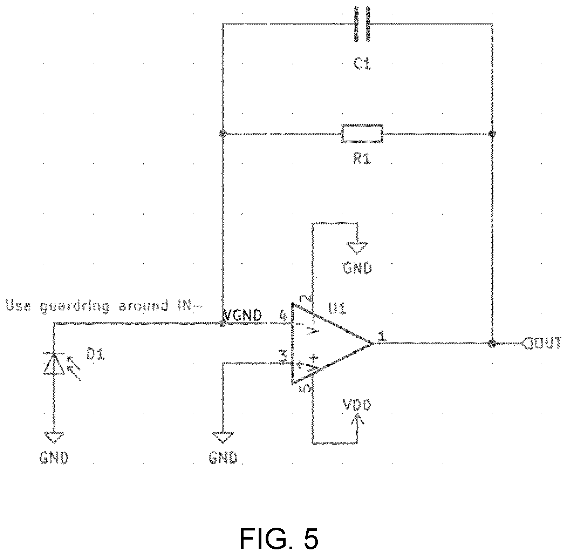

Amplifying circuits (e.g., located above each photo detector 13a in a direction along the Y-axis in Fig. 3) may be used with each photo detector 13a to convert the generated analog signal into a signal range compatible with an analog to digital converter (ADC). Based on the specifications of the photo detectors and of the ADC, these amplifying circuits may however be omitted. One such amplifying circuit is exemplified in Fig. 5. In this circuit an operational amplifier with an RC-filtered negative feedback loop is used to controllably amplify and filter the signal obtained by the photo detector 13a.

An output signal of the ADC may be the first measurement signal (or lighting measurement signal). Using a multichannel ADC allows simultaneous sampling of the amplified signals outputted by the photo detectors of the light measuring unit 13a. Alternatively, the signals of the photo detectors may be converted individually e.g., one after the other, while one LED remains activated.

Further, as shown in Fig. 3, there may be provided a peripheric measuring unit constituted by a temperature sensor 15a and/or an inertial measurement unit (IMU) 16a.

The temperature sensor 15a may be configured to measure the body/skin temperature of the user. To improve this temperature measurement, the temperature sensor 15a may be thermally decoupled from the board of the determining device 1a. If the temperature sensor 15a is not directly in contact with the user's body, it may be thermally coupled to the housing (not shown) of the determining device 1a, wherein the housing may be produced from a thermally conductive material. The thermal coupling to the housing may be achieved by a thermally conductive pad, paste, adhesive or cement.

The IMU 16a may be capable of measuring nine degrees of freedom (9-DoF) or less. Such a 9-DoF IMU 16a may be constituted by a 3D accelerometer, a 3D gyroscope and a 3D magnetometer. As a result, user activity and movement can be taken into account for the measurements. After all, organs including the urinary bladder are affected by a gravity force based on the orientation of the user's body. Therefore, differences in measurements caused by e.g., lying down, standing up or sitting, again causing the urinary bladder to change shape and/or position within the pelvic region, can be compensated by the third measurement signal (or peripheric measurement signal) outputted by the IMU 16a. Further, activities performed by the user such as walking or running can affect the urinary bladder causing differences in measurements by the first and second measurement signals (or lighting or voltage measurement signals). By obtaining changes in temperature of the user's body and motion information of the user's body, measurements obtained from the light measuring unit 13 (or 13a) and voltage measuring unit 14 (or 14a) can be compensated to improve the determination of the filling level of the urinary bladder of the user.

The IMU 16a may be attached to the board of the determining device 1a. To avoid measuring the natural frequency of the board of the determining device 1a, the IMU 16a may also be mechanically coupled to the housing (not shown) of the determining device 1a. The mechanical coupling may be achieved by adhering or friction-fitting the IMU 16a to the housing.

Further, as shown in Fig. 3, there may be provided a power circuitry 17a constituted by DC-DC converter and/or an LED driver. Herein, the DC-DC converter may be connected to the terminals of a rechargeable battery (e.g., battery 18a in Fig. 4) and provide a stable operating voltage between 1.8 V to 3.3 V for integrated circuits (iCs) of the determining device 1a. The DC-DC converter may also output larger power supply voltages (e.g., of 5 V) for driving the LEDs. The LED driver may be directly connected to the battery 18a or to the DC-DC converter and may limit the maximum current drawn by the LEDs of the light source 11a. Further LEDs may also be provided on the board of the determining device 1a to provide feedback on the internal state of the determining device 1a. Herein one or more RC-filters may be provided with the power circuitry 17a to dampen noise or ripple, e.g., induced by the switching power electronic components.

Further, as shown in Fig. 4, there may be provided (current electrode) terminals 121a used by the current source 12a to output currents through each of the (current) electrodes 121 that may be connected to the (current electrode) terminals 121a, and there may be provided (voltage electrode) terminals 141a used by the voltage measuring unit 14a to measure a voltage by use of the (voltage) electrodes 141 connected to the (voltage electrode) terminals 141a. It is worth mentioning that the (current electrode) terminals 121a and the (voltage electrode) terminals 141a may also be the same terminals 121a/141a and may be used by the current source 12a and the voltage measuring unit 14a. In this case, the operation of the current source 12a and the voltage measuring unit 14a may be controlled such that the current source 12a and the voltage measuring unit 14a do not disturb each other (e.g., by not using terminals over which current is outputted for voltage measuring and vice versa). Herein, current electrodes 121 and voltage electrodes 141 may be the same kind of electrodes that may be shared by the current source 12a and the voltage measuring unit 14a, too. This means, terminals 121a/141a may be connected to both the current source 12a and the voltage measuring unit 14a. To simplify the circuitry, however, it may be preferrable to use designated (current electrode) terminals 121a and the (voltage electrode) terminals 141a, connected to only the current source 12a and the voltage measuring unit 14a, respectively.

During operation the current source 12a and the voltage measuring unit 14a may operate without interfering with each other. This may be achieved by the current source 12a outputting a current to two terminals 121a whilst the voltage measuring unit 14a measures the voltage across two terminals 141a, at least one of which being different to the two terminals 121a.

Also, the voltage measuring unit 14a may be provided with voltage measuring pins capable of being set into a tri-state of "high impedance". Thereby voltages at all terminals 141a may be measured regardless of which terminal 121a is used for current output.

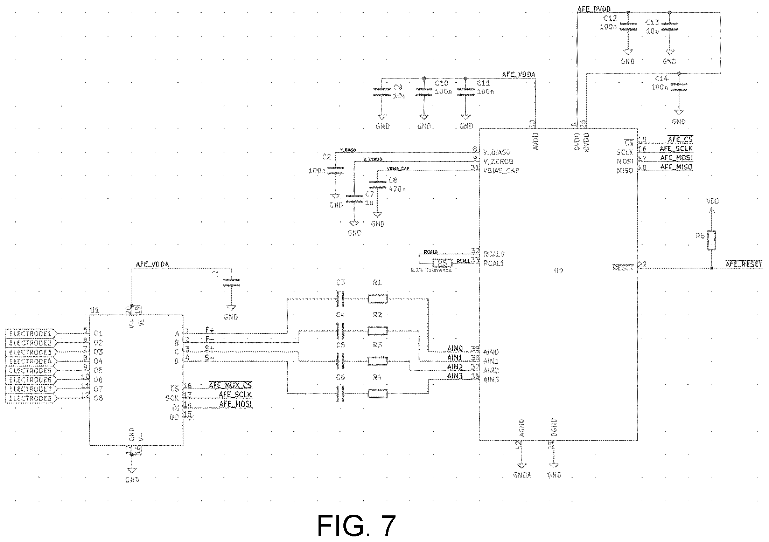

Fig. 7 and 8 illustrate examples of the analog front end of the voltage measuring unit 14a. Herein, a 4-wire body impedance analysis (BIA) approach is exemplified that uses a high precision, AC voltage source to excite a sensor with a known AC voltage (VAC). Simultaneously, a common-mode voltage is applied across the sensor. To calculate the impedance, a current (I) that flows through the unknown impedance and the voltage across the unknown impedance are measured. Then impedance is calculated by using the equation Z = V / I (Ohm's law). To comply with the standard IEC 60601 discrete isolating capacitors may be used that prevent the occurrence of DC voltage across the body, and a limiting resistor restricts the current flowing through the body.

The electrodes may be connected to the terminals 121a/141a by soldering or by use of detachable connectors, sockets or plugs.

As a modification to the present embodiment, the determining device 1, 1a may further comprise a housing, wherein the current electrodes 121 and/or the voltage electrodes 141 are held by the housing. That is to say, the electrodes 121/141 may be provided as part of the determining device 1a and/or may be provided as part of the housing of the determining device 1a. It is however not necessary to provide the electrodes 121/141 with the determining device 1a or its housing. Instead, the electrodes may be individually attached (e.g., by use of suction cups or sticky pads or by applying a plaster) to the user's body. The electrodes may also be provided within or as part of a wearable or portable as mentioned above to omit having to individually place the electrodes on the user's body.

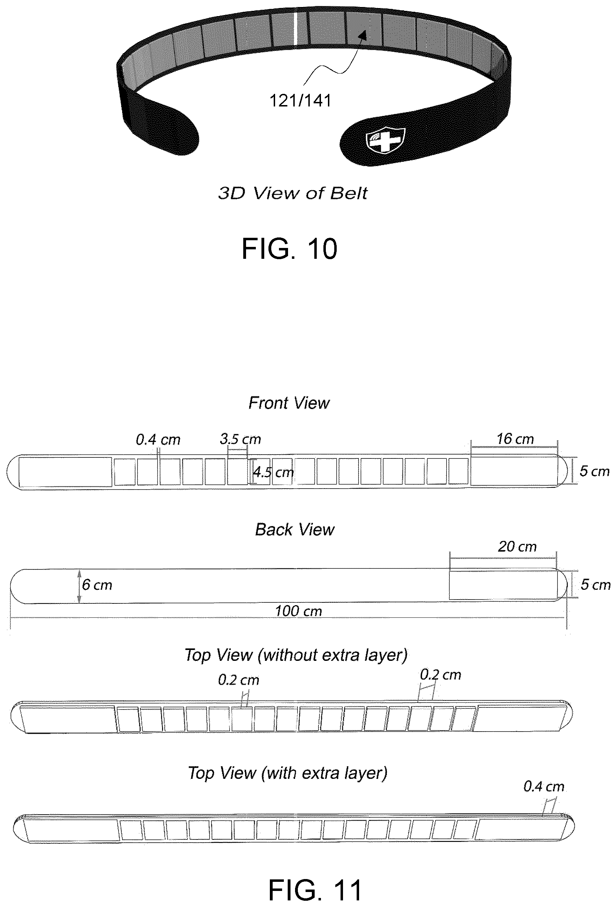

A system for determining a filling level of the urinary bladder of a user (i.e., a "determining system") is another preferred embodiment of the present disclosure. In this embodiment, the determining system comprises a determining device 1, 1a according to any of the embodiments, and means for holding the light source 11, 11a, the light measuring unit 13, 13a, and the electrodes 121, 121a, 141, 141a against the user's body. This means for holding may be any of the means discussed above for attaching the electrodes to the user's body. Herein, a wearable (e.g., a belt, underwear or T-shirt) is a preferred embodiment of the means for holding. E.g., a belt of similar dimensions as the belt illustrated in Fig. 11 may be used to hold the electrodes 121/141 at a position close to the urinary bladder of the user. Although the figure exemplifies the electrodes 121/141 as being of a rectangular shape, the electrodes 121/141 may be any conceivable shape (e.g., a circular shape or an unregular shape) and/or may be flat, protruding or receding from the belt. This means for holding may also hold the device 1, 1a itself e.g., such that the housing of the device 1, 1a may be held against the user's body.

As a modification to the present embodiment, the means for holding may be a wearable. In this case, the current electrodes 121, 121a and/or the voltage electrodes 141, 141a may be attached to or part of the wearable, and may be electrically connectable with the determining device 1, 1a.

Fig. 10 illustrates an example of a wearable as a belt with electrodes 121/141 that are to be connected to the terminals 121a/141a of the determining device 1a. As illustrated in Fig. 10, the belt may comprise 16 electrodes to also enable EIT.

For EIS however not all electrodes of this belt may be used (i.e., allowing that electrodes may be omitted from the belt). It is preferrable to use electrodes 121/141 covering a (pelvic) region of the user's body, so position the electrodes 121/141 in close proximity to the urinary bladder. Therefore, when using a belt as the wearable, the outer most electrodes 121/141 of the belt may be used for EIS. These electrodes 121/141 of the belt are located closest to the urinary bladder of the user when the belt is worn by the user. Preferrable dimensioning of the belt is illustrated in Fig. 11.

The determining device 1a may be connected to the belt (e.g., in the middle of the belt) such that the belt carries the determining device 1a. In this case, the belt should comprise means for closing it (e.g., by providing hook-and-loop fasteners to either ends of the belt, by providing a button or buckle to one end of the belt or other belt closing means). Alternatively, the determining device 1a may be used to close the belt. In this case, one or both ends of the belt may be provided with terminal connectors configured to (electrically and physically) mate with the terminals 121a/141a of the determining device 1a.

It is worth mentioning that the electrodes 121/141 need not be provided as part of the belt if the electrodes are individually attachable to the user's body or are provided as part of the determining device 1a as explained above. In this case, the wearable (e.g., belt) may only be used to carry the determining device 1a (e.g., without providing connectivity to the electrodes 121/141).

Further, as shown in Fig. 4, there may be provided the control unit 10a in the form of an IC. Some of the terminals 121a/141a may also be used for communicating to or programming the IC (e.g., the three bottom most terminals 121a/141a closest to the IC 10a).

Further, as shown in Fig. 4, there may be provided a rechargeable battery 18a which serves as a power supply for the determining device 1a. The battery may be a lithium-based pouch battery with a flat profile to minimize the thickness of the determining device 1a.

Further, as shown in Fig. 4, there may be provided a slot 19a for a memory (e.g., a microSD card). This memory may be used to record measurements and/or provide data pertaining the trained model.

Further, as shown in Fig. 4, there may be provided the evaluation unit 20a. The evaluation unit 20a may determine the filling level of the urinary bladder based on the measurement signals received from the light measuring unit 13a, the voltage measuring unit 14a and/or the peripheral measuring unit 15a/16a. The evaluation unit 20a may use the trained model to perform the determination. In this case, the trained model may be provided to the evaluation unit 20a by use of the memory in the slot 19a and/or by communicating with a computer (e.g., a smartphone).

The evaluation unit may be provided by a separate device (e.g., a computer or smartphone), connected with the determining device 1a using a communication interface. This communication interface may be wired or, preferably, wireless. In the wireless case, the evaluation unit 20a depicted in Fig. 4 may be a transceiver unit 20a (e.g., using WiFi or Bluetooth technology) communicating with a separate evaluation unit. Specifically, the transceiver unit 20a may communicate (preprocessed) measurements from the light measuring unit 13a, the voltage measuring unit 14a and/or the peripheric measuring unit 15a to the separate evaluation unit. Thereby, processing requirements and power consumption of the units mounted to the determining device 1a are reduced, maximizing the battery life of the determining device 1a.

Also, measurements may be buffered in the memory **(e.g., in** the slot 19a) and once a sufficient amount of data has been accumulated, the data may be transmitted by the transceiver unit 20a in a single transmission. Thereby, a temporary loss in connection between the transceiver unit 20a and the separate evaluation unit can be compensated since a continuous connection between the transceiver unit 20a, and the separate evaluation unit is no longer necessary.

The separate evaluation unit may be constituted by a single computer (e.g., a smartphone), a server or a group of computers (e.g., a computing center providing cloud computing) communicating with the transceiver unit 20a. This also allows more sophisticated trained models to be used that exceed the computing/storing capabilities provided by the determining device 1a.

It is also conceivable that the evaluation unit 20a depicted in Fig. 4 provides both functions of determining the filling level of the urinary bladder of the user and of communicating measurements to a separate evaluation unit. Thereby, an immediate primary determination of the filling level of the urinary bladder can be obtained without relying on computing resources external to the determination device 1a. If a connection to such external computing resources can be established, they may provide a secondary determination of the filling level of the urinary bladder (e.g., to validate the primary determination and/or to provide a more accurate determination).

It is preferrable to provide the determining device 1a as a contained system, wherein the evaluation unit 20a is provided on the PCB of the determining device 1a.

As a modification of the present embodiment, the determining device 1 may further be provided with an output unit (not shown) configured to output an alarm and/or a notification based on the determined filling level of the urinary bladder. Therefore, based on the (primary and/or secondary) determination of the filling level of the urinary bladder, feedback on the filling level can be provided to the user by use of the output unit. This output unit may be capable of outputting visual, acoustic and/or haptic signals. E.g., when the filling level reaches a predefined/critical value (e.g., 80 % if a percentage is used to indicate the filling level) and/or when (or "as soon as") a predefined timespan is remaining until the predefined level is reached a notification or an alarm may be outputted by the output unit. This notification or alarm may be: a display of a message (e.g., displayed on a screen provided to the determining device 1 or on a screen of an external computer such as a smartphone); a tune or sound (e.g., outputted by a speaker provided to the determining device 1 or provided to an external computer such as a smartphone); a vibration (e.g., generated by a vibrator provided to the determining device 1 or provided to an external computer such as a smartphone); and/or a light (e.g., emitted by a signal LED provided to the determining device 1 or achieved by flashing a screen of an external computer such as a smartphone). Herein, the notification or alarm by use of acoustic signals is preferrable for users incapable of controlling a micturition when asleep.

As a modification of the present embodiment, the determining device 1 may further be provided with an (optional) output unit (not shown) configured to output a feedback or notification regarding the positioning of the determining device 1 and/or its sensing unit/s (as explained above). This output unit may be the same or a different output unit as described above. In the latter case, the output unit may be provided to the user by use of a computer or smartphone (e.g., using audible, haptic and/or visual signaling). In either case however the user can be informed and react, if the determining device 1 and/or its sensing unit/s have been placed on the body in a sub-optimal manner.

Although illustrated as separate units in Fig. 4, the control unit 10a and evaluation unit 20a (or transceiver unit 20a in case data is communicated to an external computer as described above) may be provided within the same microcontroller package. In this case, the single microcontroller package performs the functions of: controlling the light source 11a and/or current source 12a; obtaining the first measurement signals from the light measuring unit 13a and/or the voltage measuring unit 14a; and determining the filling level of the urinary bladder based thereon (or communicating the measurement signals to a separate evaluation entity, e.g., for the secondary determination as described above).

### Reference Sign List

1, 1a: determining device
10: control unit
11, 11a, 11a-1 ... 11a-3: light source / light emitting diode
12, 12a: current source
121, 121a: current electrode
13, 13a: light measuring unit
14, 14a: voltage measuring unit
15a: peripheric measuring unit
16a: inertial measurement unit
17a-1, 17a2: power supply unit
18a: battery
19a: memory / card slot
141: voltage electrode
20, 20a: evaluation unit / communication unit

## Claims

1. A non-invasive device (1, 1a) for determining a filling level of the urinary bladder of a user, comprising:
a light source (11, 11a) adapted to irradiate near-infrared light into a region of the user's body covering the pelvic region;
a current source (12, 12a) adapted to output and pass a current through the region of the user's body using at least two electrodes (121, 121a);
a control unit (10, 10a) adapted to:
output, to the light source (11, 11a), a first control signal matched to the user, for controlling the light irradiated by the light source (11, 11a), and
output, to the current source (12, 12a), a second control signal matched to the user, for controlling the current output by the current source (12, 12a);
a light measuring unit (13, 13a) adapted to:
measure the light emitted from the light source (11, 11a) and light reflected from organs of the user's body, and
output a first measuring signal corresponding to the measured light;
a voltage measuring unit (14, 14a) adapted to:
measure a voltage based on the current passed through the region of the user's body using at least two electrodes (141, 141a) resting against the user's body, and
output a second measurement signal corresponding to the measured voltage; and
an evaluation unit (20, 20a) adapted to determine the filling level of the urinary bladder based on a selection of the first measurement signal and/or the second measurement signal, wherein the selection is based on a reliability or a usability of the light measuring unit (13, 13a) and/or the voltage measuring unit (14, 14a).

2. The device (1, 1a) according to claim 1, further comprising:
a peripheric measuring unit (15a, 16a) adapted to:
measure peripheric properties including acceleration forces, rotational movements, temperature, inclination and/or humidity, and
output a third measurement signal corresponding to the peripheric properties,
wherein the evaluation unit (20) is further adapted to determine the filling level of the urinary bladder based on the first measurement signal, the second measurement signal and/or the third measurement signal.

3. The device (1, 1a) according to claim 1 or 2, wherein
at least eight electrodes (121, 121a) are distributed over the region of the user's body; and
the second control signal controls the current source (12, 12a) to pass the current through the region of the user's body using a varying selection of two or more electrodes (121, 121a) of the at least eight electrodes (121, 121a).

4. The device (1, 1a) according to claim 3 if depending on claim 2, wherein
the voltage measuring unit (14, 14a) is adapted to:
measure voltages based on different selections of two or more electrodes (141, 141a), and
output a second measurement signals corresponding to the measured voltages; and
the evaluation unit (20, 20a) is adapted to:
compute an impedance distribution of a cross section of the user's body based on the second measurement signals, and
determine the filling level of the urinary bladder based on the first measurement signal, the second measurement signal, the third measurement signal and/or the impedance distribution.

5. The device (1, 1a) according to any one of claims 1 to 4, wherein
the light irradiated by the light source (11, 11a) is near-infrared light with a wavelength of at least 600 nm and at most 1100 nm and more preferably with a wavelength of at least 830 nm and at most 975 nm.

6. The device (1, 1a) according to any one of claims 1 to 5, wherein
the light source (11, 11a) comprises a plurality of light emitting elements configured to emit near-infrared light of 775 nm, 855 nm, 880 nm, 940 nm, 950 nm, 970 nm, 975 nm, 1020 nm and/or 1050 nm.

7. The device (1) according to any one of claims 1 to 6, further comprising a housing, wherein the electrodes (121, 121a, 141, 141a) are held by the housing.

8. The device (1) according to any one of claims 1 to 7, wherein
the evaluation unit (20, 20a) is configured to select the first measuring signal, the second measuring signal, the third measuring signal and/or the impedance distribution based on a parameter,
the parameter indicates the reliability or usability of the light measuring unit (13, 13a), the voltage measuring unit (14, 14a) and/or peripheric measuring unit (15a, 16a).

9. The device (1) according to any one of claims 1 to 8, wherein
the control unit (10, 10a) is configured to adjust the first control signal and/or the second control signal based on a parameter,
the parameter indicates the reliability or usability of the light measuring unit (13, 13a) and/or the voltage measuring unit (14, 14a).

10. The device (1) according to any one of claims 1 to 9, wherein
the evaluation unit (20, 20a) is configured to determine an accuracy of a positioning of the determining device (1) and/or of a sensing unit on the body of the user by using the first measuring signal, the second measuring signal, the third measuring signal and/or the impedance distribution;
the sensing unit is the light source (11, 11a), the electrodes (121, 121a, 141, 141a) and/or the light measuring unit (13, 13a).

11. The device (1) according to claim 10, wherein
the evaluation unit (20, 20a), to determine the accuracy of the positioning of the determining device (1) and/or the sensing unit, is configured
to compare a reference measurement obtained from the light measuring unit (13, 13a), the voltage measuring unit (14, 14a) and/or the peripheric measuring unit (15a, 16a) with the first measuring signal, the second measuring signal, the third measuring signal and/or the impedance distribution, and/or
to:
detect an anatomic marker based the first measuring signal, the second measuring signal, the third measuring signal and/or the impedance distribution,
determine a position of the determining device (1) and/or the sensing unit on the body of the user from the location to the anatomic marker, and
compare the determined position with a predetermined ideal position.

12. The device (1) according to any one of claims 1 to 11, further provided with an output unit configured to output an alarm and/or a notification based on the determined filling level of the urinary bladder and/or based on the accuracy of the positioning of the determining device (1) and/or the sensing unit.

13. A computer-implemented method for producing a trained model capable of determining a filling level of the urinary bladder of a user, the method comprising:
applying a device (1, 1a) according to claim 4 and any claim depending thereon to the region of the user's body;
obtaining time series data based on the first measurement signal, the second measurement signal, the third measurement signal and/or the impedance distribution;
obtaining a notification of a predetermined event;
estimating a filling level of the urinary bladder based on the time series data and the notification of the predetermined event; and
training the model to:
associate the time series data with the filling level of the urinary bladder, and
predict the filling level of the urinary bladder and/or a progress of the filling level of the urinary bladder based on the first measurement signal, the second measurement signal, the third measurement signal and/or the impedance distribution.

14. The method according to claim 13, wherein
the filling level of the urinary bladder is indicative of the filling level of the urinary bladder by use of a classification; and
the classification is preferably a binary classification, a multi-level classification, a percentage classification and/or a volumetric classification of the filling level of the urinary bladder.

15. The device (1, 1a) according to any one of claims 1 to 12, wherein the evaluation unit (20) is adapted to use a model trained according to claim 13 or 14 to determine the filling level of the urinary bladder.

16. A system for determining a filling level of the urinary bladder of a user, the system comprising:
a device (1, 1a) according to any one of claims 1 to 12 and 15; and
means for holding the light source (11, 11a), the light measuring unit (13, 13a) and the electrodes (121, 121a, 141, 141a) against the user's body.

17. The system according to claim 16, wherein
the means for holding is a wearable, such as a belt, an underwear or T-shirt, and
the electrodes (121, 121a, 141, 141a) are attached to or part of a wearable, and
the electrodes (121, 121a, 141, 141a) are electrically connectable with the device (1, 1a).

## Patentansprüche

1. Nichtinvasive Vorrichtung (1, 1a) zur Bestimmung eines Blasenfüllstands eines Benutzers, umfassend:
eine Lichtquelle (11, 11a), die dazu angepasst ist, Nahinfrarotlicht in eine Region des Körpers des Benutzers abzustrahlen, der die Beckenregion bedeckt;
eine Stromquelle (12, 12a), die dazu angepasst ist, unter Verwendung von mindestens zwei Elektroden (121, 121a) einen Strom auszugeben und durch die Körperregion des Benutzers zu leiten;
eine Steuereinheit (10, 10a), die angepasst ist zum:
Ausgeben eines ersten, auf den Benutzer abgestimmten Steuersignals an die Lichtquelle (11, 11a) zum Steuern des von der Lichtquelle (11, 11a) abgestrahlten Lichts, und
Ausgeben eines zweiten, auf den Benutzer abgestimmten Steuersignals an die Stromquelle (12, 12a) zum Steuern der von der Stromquelle (12, 12a) abgegebenen Stromstärke;
eine Lichtmesseinheit (13, 13a), die angepasst ist zum:
Messen des von der Lichtquelle (11, 11a) emittierten Lichts und von den Organen des Körpers des Benutzers reflektiertem Licht, und
Ausgeben eines ersten Messsignals, das dem gemessenen Licht entspricht;
eine Spannungsmesseinheit (14, 14a), die angepasst ist zum:
Messen einer Spannung basierend auf dem durch die Körperregion des Benutzers fließenden Strom unter Verwendung von mindestens zwei Elektroden (141, 141a), die am Körper des Benutzers anliegen, und
Ausgeben eines zweiten Messsignals, das der gemessenen Spannung entspricht; und
eine Auswerteeinheit (20, 20a), die dazu angepasst ist, den Blasenfüllstand basierend auf einer Auswahl des ersten Messsignals und/oder des zweiten Messsignals zu bestimmen, wobei die Auswahl auf einer Zuverlässigkeit oder einer Verwendbarkeit der Lichtmesseinheit (13, 13a) und/oder der Spannungsmesseinheit (14, 14a) basiert.

2. Vorrichtung (1, 1a) nach Anspruch 1, weiter umfassend:
eine periphere Messeinheit (15a, 16a), die angepasst ist zum:
Messen peripherer Eigenschaften, einschließlich Beschleunigungskräften, Rotationsbewegungen, Temperatur, Neigung und/oder Feuchtigkeit, und
Ausgeben eines dritten Messsignals, das den peripheren Eigenschaften entspricht,
wobei die Auswerteeinheit (20) weiter dazu angepasst ist, den Blasenfüllstand basierend auf dem ersten Messsignal, dem zweiten Messsignal und/oder dem dritten Messsignal zu bestimmen.

3. Vorrichtung (1, 1a) nach Anspruch 1 oder 2, wobei
mindestens acht Elektroden (121, 121a) über den Körperbereich des Benutzers verteilt sind; und
das zweite Steuersignal die Stromquelle (12, 12a) steuert, um den Strom durch die Körperregion des Benutzers zu leiten, wobei eine variierende Auswahl von zwei oder mehr Elektroden (121, 121a) der mindestens acht Elektroden (121, 121a) verwendet wird.

4. Vorrichtung (1, 1a) nach Anspruch 3, falls abhängig von Anspruch 2, wobei
die Spannungsmesseinheit (14, 14a) angepasst ist zum:
Messen von Spannungen basierend auf verschiedenen Auswahlen zweier oder mehrerer Elektroden (141, 141a), und
Ausgeben eines zweiten Messsignals, das den gemessenen Spannungen entspricht; und
die Auswerteeinheit (20, 20a) angepasst ist zum:
Berechnen einer Impedanzverteilung eines Querschnitts des Körpers des Benutzers basierend auf den zweiten Messsignalen, und
Bestimmen des Blasenfüllstands basierend auf dem ersten Messsignal, dem zweiten Messsignal, dem dritten Messsignal und/oder der Impedanzverteilung.

5. Vorrichtung (1, 1a) nach einem der Ansprüche 1 bis 4, wobei
das von der Lichtquelle (11, 11a) abgestrahlte Licht Nahinfrarotlicht mit einer Wellenlänge von mindestens 600 nm und höchstens 1100 nm und besonders bevorzugt mit einer Wellenlänge von mindestens 830 nm und höchstens 975 nm ist.

6. Vorrichtung (1, 1a) nach einem der Ansprüche 1 bis 5, wobei
die Lichtquelle (11, 11a) eine Vielzahl von Licht emittierenden Elementen umfasst, die so konfiguriert sind, dass sie Nahinfrarotlicht von 775 nm, 855 nm, 880 nm, 940 nm, 950 nm, 970 nm, 975 nm, 1020 nm und/oder 1050 nm emittieren.

7. Vorrichtung (1) nach einem der Ansprüche 1 bis 6, weiter umfassend ein Gehäuse, wobei die Elektroden (121, 121a, 141, 141a) durch das Gehäuse gehalten werden.

8. Vorrichtung (1) nach einem der Ansprüche 1 bis 7, wobei
die Auswerteeinheit (20, 20a) dazu konfiguriert ist, das erste Messsignal, das zweite Messsignal, das dritte Messsignal und/oder die Impedanzverteilung basierend auf einem Parameter auszuwählen,
wobei der Parameter die Zuverlässigkeit oder Verwendbarkeit der Lichtmesseinheit (13, 13a), der Spannungsmesseinheit (14, 14a) und/oder der peripheren Messeinheit (15a, 16a) angibt.

9. Vorrichtung (1) nach einem der Ansprüche 1 bis 8, wobei
die Steuereinheit (10, 10a) dazu konfiguriert ist, das erste Steuersignal und/oder das zweite Steuersignal basierend auf einem Parameter anzupassen,
wobei der Parameter die Zuverlässigkeit oder Verwendbarkeit der Lichtmesseinheit (13, 13a) und/oder der Spannungsmesseinheit (14, 14a) angibt.

10. Vorrichtung (1) nach einem der Ansprüche 1 bis 9, wobei
die Auswerteeinheit (20, 20a) dazu konfiguriert ist, eine Genauigkeit einer Positionierung der Bestimmungsvorrichtung (1) und/oder einer Sensoreinheit am Körper des Benutzers unter Verwendung des ersten Messsignals, des zweiten Messsignals, des dritten Messsignals und/oder der Impedanzverteilung zu bestimmen;
die Sensoreinheit die Lichtquelle (11, 11a), die Elektroden (121, 121a, 141, 141a) und/oder die Lichtmesseinheit (13, 13a) ist.

11. Vorrichtung (1) nach Anspruch 10, wobei
die Auswerteeinheit (20, 20a) zum Bestimmen der Genauigkeit der Positionierung der Bestimmungsvorrichtung (1) und/oder der Sensoreinheit dazu konfiguriert ist,
eine von der Lichtmesseinheit (13, 13a), der Spannungsmesseinheit (14, 14a) und/oder der peripheren Messeinheit (15a, 16a) erhaltene Referenzmessung mit dem ersten Messsignal, dem zweiten Messsignal, dem dritten Messsignal und/oder der Impedanzverteilung zu vergleichen, und/oder
zum:
Detektieren eines anatomischen Markers basierend auf dem ersten Messsignal, dem zweiten Messsignal, dem dritten Messsignal und/oder der Impedanzverteilung,
Bestimmen einer Position der Bestimmungsvorrichtung (1) und/oder der Sensoreinheit am Körper des Benutzers vom Standort bis zur anatomischen Markierung, und
Vergleichen der bestimmten Position mit einer vorgegebenen Idealposition.

12. Vorrichtung (1) nach einem der Ansprüche 1 bis 11, weiter bereitgestellt mit einer Ausgabeeinheit, die dazu konfiguriert ist, einen Alarm und/oder eine Benachrichtigung basierend auf dem bestimmten Blasenfüllstand und/oder basierend auf der Genauigkeit der Positionierung der Bestimmungsvorrichtung (1) und/oder der Sensoreinheit auszugeben.

13. Computerimplementiertes Verfahren zum Erstellen eines trainierten Modells, das in der Lage ist, den Blasenfüllstand eines Benutzers zu bestimmen, wobei das Verfahren umfasst:
Anbringen einer Vorrichtung (1, 1a) nach Anspruch 4 und einem beliebigen davon abhängigen Anspruch an der Körperregion des Benutzers;
Erhalten von Zeitreihendaten basierend auf dem ersten Messsignal, dem zweiten Messsignal, dem dritten Messsignal und/oder der Impedanzverteilung;
Erhalten einer Benachrichtigung über ein vorbestimmtes Ereignis;
Schätzen eines Blasenfüllstands basierend auf den Zeitreihendaten und der Benachrichtigung über das vorbestimmte Ereignis; und
Trainieren des Modells zum:
Verknüpfen der Zeitreihendaten mit dem Blasenfüllstand, und
Vorhersagen des Blasenfüllstands und/oder einer Prognose des Blasenfüllstands basierend auf dem ersten Messsignal, dem zweiten Messsignal, dem dritten Messsignal und/oder der Impedanzverteilung.

14. Verfahren nach Anspruch 13, wobei
der Blasenfüllstand unter Verwendung einer Klassifizierung den Blasenfüllstand angibt; und
die Klassifizierung vorzugsweise eine binäre Klassifizierung, eine mehrstufige Klassifizierung, eine prozentuale Klassifizierung und/oder eine volumetrische Klassifizierung des Blasenfüllstands ist.

15. Vorrichtung (1, 1a) nach einem der Ansprüche 1 bis 12, wobei die Auswerteeinheit (20) dazu angepasst ist, zum Bestimmen des Blasenfüllstands ein nach Anspruch 13 oder 14 trainiertes Modell zu verwenden.

16. System zur Bestimmung eines Blasenfüllstands eines Benutzers, wobei das System umfasst:
eine Vorrichtung (1, 1a) nach einem der Ansprüche 1 bis 12 und 15; und
Mittel zum Halten der Lichtquelle (11, 11a), der Lichtmesseinheit (13, 13a) und der Elektroden (121, 121a, 141, 141a) am Körper des Benutzers.

17. System nach Anspruch 16, wobei
die Mittel zum Halten ein tragbares Objekt, wie beispielsweise ein Gürtel, eine Unterwäsche oder ein T-Shirt sind, und
die Elektroden (121, 121a, 141, 141a) an einem tragbaren Gerät befestigt sind oder Teil davon sind, und
die Elektroden (121, 121a, 141, 141a) mit der Vorrichtung (1, 1a) elektrisch verbindbar sind.

## Revendications

1. Dispositif non invasif (1, 1a) pour déterminer un niveau de remplissage de la vessie urinaire d'un utilisateur, comprenant :
une source de lumière (11, 11a) conçue pour émettre de la lumière proche infrarouge dans une région du corps de l'utilisateur couvrant la région pelvienne ;
une source de courant (12, 12a) conçue pour délivrer et faire passer un courant à travers la région du corps de l'utilisateur en utilisant au moins deux électrodes (121, 121a) ;
une unité de commande (10, 10a) conçue pour :
émettre, vers la source de lumière (11, 11a), un premier signal de commande adapté à l'utilisateur, pour commander la lumière émise par la source de lumière (11, 11a), et
émettre, vers la source de courant (12, 12a), un second signal de commande adapté à l'utilisateur, pour commander la sortie de courant par la source de courant (12, 12a) ;
une unité de mesure de lumière (13, 13a) conçue pour :
mesurer la lumière émise par la source de lumière (11, 11a) et la lumière réfléchie par les organes du corps de l'utilisateur, et
émettre un premier signal de mesure correspondant à la lumière mesurée ;
une unité de mesure de tension (14, 14a) conçue pour :
mesurer une tension sur la base du courant traversant la région du corps de l'utilisateur à l'aide d'au moins deux électrodes (141, 141a) reposant sur le corps de l'utilisateur, et
émettre un deuxième signal de mesure correspondant à la tension mesurée ; et
une unité d'évaluation (20, 20a) conçue pour déterminer le niveau de remplissage de la vessie urinaire sur la base d'une sélection du premier signal de mesure et/ou du deuxième signal de mesure, dans lequel la sélection est basée sur une fiabilité ou une exploitabilité de l'unité de mesure de lumière (13, 13a) et/ou de l'unité de mesure de tension (14, 14a).

2. Dispositif (1, 1a) selon la revendication 1, comprenant en outre :
une unité de mesure périphérique (15a, 16a) conçue pour :
mesurer des propriétés périphériques incluant des forces d'accélération, des mouvements de rotation, la température, l'inclinaison et/ou l'humidité, et
émettre un troisième signal de mesure correspondant aux propriétés périphériques,
dans lequel l'unité d'évaluation (20) est en outre conçue pour déterminer le niveau de remplissage de la vessie urinaire sur la base du premier signal de mesure, du deuxième signal de mesure et/ou du troisième signal de mesure.

3. Dispositif (1, 1a) selon la revendication 1 ou la revendication 2, dans lequel
au moins huit électrodes (121, 121a) sont réparties sur la région du corps de l'utilisateur ; et
le second signal de commande commande la source de courant (12, 12a) pour faire passer le courant à travers la région du corps de l'utilisateur en utilisant une sélection variable de deux électrodes (121, 121a) ou plus parmi les au moins huit électrodes (121, 121a).

4. Dispositif (1, 1a) selon la revendication 3, si elle dépend de la revendication 2, dans lequel
l'unité de mesure de tension (14, 14a) est conçue pour :
mesurer des tensions sur la base de différentes sélections de deux électrodes (141, 141a) ou plus, et
émettre un deuxième signal de mesure correspondant aux tensions mesurées ; et
l'unité d'évaluation (20, 20a) est conçue pour :
calculer une distribution d'impédance d'une section transversale du corps de l'utilisateur sur la base des deuxièmes signaux de mesure, et
déterminer le niveau de remplissage de la vessie urinaire sur la base du premier signal de mesure, du deuxième signal de mesure, du troisième signal de mesure et/ou de la distribution d'impédance.

5. Dispositif (1, 1a) selon l'une quelconque des revendications 1 à 4, dans lequel
la lumière émise par la source de lumière (11, 11a) est de la lumière proche infrarouge d'une longueur d'onde d'au moins 600 nm et d'au plus 1100 nm et, plus préférentiellement, d'une longueur d'onde d'au moins 830 nm et d'au plus 975 nm.

6. Dispositif (1, 1a) selon l'une quelconque des revendications 1 à 5,
dans lequel
la source de lumière (11, 11a) comprend une pluralité d'éléments émetteurs de lumière configurés pour émettre une lumière proche infrarouge de 775 nm, 855 nm, 880 nm, 940 nm, 950 nm, 970 nm, 975 nm, 1020 nm et/ou 1050 nm.

7. Dispositif (1) selon l'une quelconque des revendications 1 à 6, comprenant en outre un boîtier, dans lequel les électrodes (121, 121a, 141, 141a) sont maintenues par le boîtier.

8. Dispositif (1) selon l'une quelconque des revendications 1 à 7, dans lequel
l'unité d'évaluation (20, 20a) est configurée pour sélectionner le premier signal de mesure, le deuxième signal de mesure, le troisième signal de mesure et/ou la distribution d'impédance sur la base d'un paramètre,
le paramètre indique la fiabilité ou l'exploitabilité de l'unité de mesure de lumière (13, 13a), de l'unité de mesure de tension (14, 14a) et/ou de l'unité de mesure périphérique (15a, 16a).

9. Dispositif (1) selon l'une quelconque des revendications 1 à 8, dans lequel
l'unité de commande (10, 10a) est configurée pour ajuster le premier signal de commande et/ou le second signal de commande sur la base d'un paramètre,
le paramètre indique la fiabilité ou l'exploitabilité de l'unité de mesure de lumière (13, 13a) et/ou de l'unité de mesure de tension (14, 14a).

10. Dispositif (1) selon l'une quelconque des revendications 1 à 9, dans lequel
l'unité d'évaluation (20, 20a) est configurée pour déterminer une précision d'un positionnement du dispositif (1) de détermination et/ou d'une unité de détection sur le corps de l'utilisateur en utilisant le premier signal de mesure, le deuxième signal de mesure, le troisième signal de mesure et/ou la distribution d'impédance ;
l'unité de détection est la source de lumière (11, 11a), les électrodes (121, 121a, 141, 141a) et/ou l'unité de mesure de lumière (13, 13a).

11. Dispositif (1) selon la revendication 10, dans lequel
l'unité d'évaluation (20, 20a), pour déterminer la précision du positionnement du dispositif (1) de détermination et/ou de l'unité de détection, est configurée
pour comparer une mesure de référence obtenue à partir de l'unité de mesure de lumière (13, 13a), de l'unité de mesure de tension (14, 14a) et/ou de l'unité de mesure périphérique (15a, 16a) avec le premier signal de mesure, le deuxième signal de mesure, le troisième signal de mesure et/ou la distribution d'impédance, et/ou
pour :
détecter un repère anatomique sur la base du premier signal de mesure, du deuxième signal de mesure, du troisième signal de mesure et/ou de la distribution d'impédance,
déterminer une position du dispositif (1) de détermination et/ou de l'unité de détection sur le corps de l'utilisateur de l'emplacement au repère anatomique, et
comparer la position déterminée avec une position idéale prédéterminée.

12. Dispositif (1) selon l'une quelconque des revendications 1 à 11, doté en outre d'une unité de sortie configurée pour émettre une alarme et/ou une notification sur la base du niveau de remplissage déterminé de la vessie urinaire et/ou sur la base de la précision du positionnement du dispositif de détermination (1) et/ou de l'unité de détection.

13. Procédé mis en œuvre par ordinateur pour produire un modèle formé apte à déterminer un niveau de remplissage de la vessie urinaire d'un utilisateur, le procédé comprenant :
l'application d'un dispositif (1, 1a), selon la revendication 4 et une quelconque revendication dépendant de celle-ci, sur la région du corps de l'utilisateur ;
l'obtention de données chronologiques sur la base du premier signal de mesure, du deuxième signal de mesure, du troisième signal de mesure et/ou de la distribution d'impédance ;
l'obtention d'une notification d'un événement prédéterminé ;
l'estimation d'un niveau de remplissage de la vessie urinaire sur la base des données chronologiques et de la notification de l'événement prédéterminé ; et
la formation du modèle pour :
associer les données chronologiques au niveau de remplissage de la vessie urinaire, et
prédire le niveau de remplissage de la vessie urinaire et/ou une progression du niveau de remplissage de la vessie urinaire sur la base du premier signal de mesure, du deuxième signal de mesure, du troisième signal de mesure et/ou de la distribution d'impédance.

14. Procédé selon la revendication 13, dans lequel
le niveau de remplissage de la vessie urinaire indique le niveau de remplissage de la vessie urinaire au moyen d'une classification ; et
la classification est de préférence une classification binaire, une classification à plusieurs niveaux, une classification en pourcentage et/ou une classification volumétrique du niveau de remplissage de la vessie urinaire.

15. Dispositif (1, 1a) selon l'une quelconque des revendications 1 à 12, dans lequel l'unité d'évaluation (20) est conçue pour utiliser un modèle formé selon la revendication 13 ou la revendication 14 pour déterminer le niveau de remplissage de la vessie urinaire.

16. Système de détermination d'un niveau de remplissage de la vessie urinaire d'un utilisateur, le système comprenant :
un dispositif (1, 1a) selon l'une quelconque des revendications 1 à 12 et la revendication 15 ; et
un moyen pour maintenir la source de lumière (11, 11a), l'unité de mesure de lumière (13, 13a) et les électrodes (121, 121a, 141, 141a) contre le corps de l'utilisateur.

17. Système selon la revendication 16, dans lequel
le moyen destiné au maintien est un élément à porter sur soi, tel qu'une ceinture, un sous-vêtement ou un t-shirt, et
les électrodes (121, 121a, 141, 141a) sont fixées à ou font partie d'un élément à porter sur soi, et
les électrodes (121, 121a, 141, 141a) peuvent être connectées électriquement au dispositif (1, 1a).
